# EUROPEAN PATENT APPLICATION

(11) **EP 4 778 461 A1**
(43) Date of publication of application: **22.07.2026**
(21) Application number: 25170459.9
(22) Date of filing: 14.04.2025
(51) Int. Cl.: A61B 5/257, A61B 5/00

(54) **WEARABLE DEVICE INCLUDING STRUCTURE FOR PREVENTING NOISE CAUSED BY STATIC ELECTRICITY**

(30) Priority: 16.01.2025 KR 20250006609
(71) Applicant: Atsens Co., Ltd., Seongnam-si Gyeonggi-do 13590 (KR)
(72) Inventor: CHO, Seung Bum, Gwacheon-si, Gyeonggi-do (KR); JEONG, Jong Ook, Bundang-gu, Seongnam-si Gyeonggi-do (KR); PARK, Soon Keun, Giheung-gu, Yongin-si, Gyeonggi-do (KR)
(74) Representative: Nederlandsch Octrooibureau

(57) **Abstract**

A wearable device is provided. The wearable device is used by being attached to a user's skin. The wearable device includes a main body unit having a housing and a substrate, the substrate being arranged inside the housing, an electrode unit including a sensing electrode connected to the main body unit, and a patch pad unit including one or more conductive members, the one or more conductive members being configured to electrically connect the electrode unit to the user's skin. The electrode unit includes a shielding layer that is not electrically connected to the main body unit. The shielding layer is conductive with a floating potential.

## Description

### CROSS-REFERENCE TO RELATED APPLICATION

This application is based on and claims priority under 35 U.S.C. §119 to Korean Patent Application No. 10-2025-0006609, filed on January 16, 2025, in the Korean Intellectual Property Office, the disclosure of which is incorporated by reference herein in its entirety.

### BACKGROUND

### 1. Field

The disclosure relates to a wearable device, and more particularly, to a wearable device including a structure for preventing noise from deteriorating a measurement signal due to static electricity.

### 2. Description of the Related Art

A wearable device may be attached to a user's body, such as the user's abdomen, back, shoulder, or arm, to measure biosignals such as electromyography, electrocardiogram, pulse signals related to blood pressure, respiratory signals, or brain waves. For example, a wearable device for measuring an electrocardiogram, whose electrodes are in contact with the user's skin, detects electrical activity in the user's heart, and measures the electrocardiogram. Due to such characteristics, the wearable device should operate while being attached to the user's body for a long time.

Meanwhile, on dry days like in winter, static electricity is easily generated due to friction between clothes and the user's body. In particular, when the user does activities while the wearable device is attached to his or her body, static electricity is inevitably generated due to friction between clothes and the wearable device or the user's body. The generated static electricity generates noise in a measurement signal of the wearable device and thus deteriorates the measurement precision.

The above background art is technical information possessed by the inventor to derive the disclosure or obtained during a process of deriving the disclosure, and is not necessarily considered to be known art open to the general public prior to the filing of the disclosure.

A wearable device may be attached to a user's body, such as the user's abdomen, back, shoulder, or arm, to measure bio-signals such as electromyography, electrocardiogram, electrooculography, electroencephalography (EEG), pulse signals related to blood pressure, respiratory signals, or brain waves.

Electroencephalography (EEG) records the brain's electrical activity, and the wearable device for measuring electroencephalography can be attached to the user's scalp to measure the brainwave activity.

Electromyography (EMG) records the electrical activity of muscles and is used to analyze muscle function, health status, or interactions between nerves and muscles. The wearable device for measuring electromyography is attached to the skin surface to detect the electrical activity in the muscles.

The wearable device for measuring electrocardiogram (ECG) is attached to the user's body so that electrodes of the wearable device are in contact with the skin, thereby detecting the electrical activity in the heart and measuring the electrocardiogram.

Electrooculography (EOG) records electrical signals related to eyes to measure eye movements and can be obtained by measuring the potential difference between the cornea and the retina. The wearable device for measuring electrooculography is attached near each eye to detect electrical signals, calculate the potential difference, and measure electrooculography.

The wearable device is attached to the skin at a predetermined location based on the type of signals to be measured to detect electrical signals. To ensure accurate diagnosis, the wearable device must remain attached to the user's body for at least 24 hours and up to 14 days to continuously detect the electrical signals.

However, the wearable device has a disadvantage in that during prolonged measurement periods, the wearable device cannot measure signals when adhesion to the skin is not maintained.

Additionally, when the user wears clothing and moves in a state in which the wearable device is attached to the user's body, static electricity generated by the clothing and movement may introduce noise into the electrical signals.

To address the disadvantages, there is a growing need for a wearable device that enhances adhesion to the skin and reduces the effects of external environmental factors.

### SUMMARY

The disclosure has been devised to solve the above technical problem and provides a wearable device including a structure for preventing noise caused by a static electricity.

However, such a technical problem is an example, and the objective of disclosure to solve is not limited thereto.

Additional aspects will be set forth in part in the description which follows and, in part, will be apparent from the description, or may be learned by practice of the presented embodiments of the disclosure.

According to an embodiment of the disclosure, a wearable device, which is used by being attached to a user's skin, includes a main body unit including a housing and a substrate, the substrate being arranged inside the housing, an electrode unit including a sensing electrode connected to the main body unit, and a patch pad unit including one or more conductive members, the one or more conductive members being configured to electrically connect the electrode unit to the user's skin. The electrode unit includes a shielding layer not to be electrically connected to the main body unit, the shielding layer being conductive with a floating potential.

According to an embodiment of the disclosure, in the wearable device, the shielding layer may be arranged on an upper surface of the electrode unit to cover an electrode pattern layer.

According to an embodiment of the disclosure, in the wearable device, the shielding layer may include a first shielding region and a second shielding region, the first shielding region being spaced apart from an edge of the main body unit by a certain distance, and the second shielding region extending from both sides of the first shielding region.

According to an embodiment of the disclosure, in the wearable device, when viewed from a top, a connection portion of the sensing electrode and the main body unit may be arranged inside the first shielding region, and the one or more conductive members may be arranged inside the second shielding region.

According to an embodiment of the disclosure, in the wearable device, the second shielding region may have a mesh structure therein.

According to an embodiment of the disclosure, in the wearable device, the shielding layer may be printed on an upper surface of the electrode unit with a conducitve ink.

According to an embodiment of the disclosure, in the wearable device, a certain potential may be applied to the shielding layer so that external static electricity does not flow into the main body unit.

According to an embodiment of the disclosure, in the wearable device, the electrode unit may further include an electrode pattern layer that is printed on a rear surface of the electrode unit, is in contact with the user's skin through the one or more conductive members, and is connected to the shielding layer to cause external static electricity to flow into the user's skin.

According to an embodiment of the disclosure, a wearable device, which is used by being attached to a user's skin, includes: a main body unit including a housing and a substrate, the substrate being arranged inside the housing; an electrode unit including a sensing electrode connected to the main body unit; and a patch pad unit including one or more conductive members, the one or more conductive members being configured to electrically connect the electrode unit to the user's skin, wherein the electrode unit includes a conductive shielding layer including an electrode that is not electrically connected to the main body unit and is connected to the user's skin.

To accomplish the above object, according to the present invention, there is provided a wearable device including: a main body unit which includes a housing and a processing unit arranged within the housing; and an electrode unit which is electrically connected to the main body unit, includes at least one sensing electrode and electrical circuits for the at least one sensing electrode, and is formed of a conductive member, wherein the electrode unit includes a shielding layer having a predetermined pattern formed in a certain region and a bottom layer getting in contact with a user's skin, and the shielding layer is laminated or coated with a predetermined pattern made of an opaque material to diffuse external noise.

The shielding layer includes a first region having the boundary where the main body unit is positioned and a first pattern, and a second region excluding the first region and having a second pattern.

On the shielding layer, a predetermined pattern is formed in the region where the main body unit is not positioned.

The first pattern formed on the shielding layer is a solid pattern that increases bonding strength with the main body unit.

The second pattern formed on the shielding layer is a mesh pattern that reduces external signals including static electricity caused by a user's clothing.

An electrode that senses electro-physical signals (electrocardiogram, electromyography, electrooculography, and impedance) is arranged in an opening of the bottom layer.

The electrode that detects capacitance is arranged in the opening of the bottom layer.

Through the opening of the bottom layer, the sensing electrodes, an insulator, and an air layer are vertically arranged to electrically sense the distance from the skin and detect distance changes caused by skin movement as capacitance changes caused by breathing activity or heartbeat pulse.

Through the opening of the bottom layer, the sensing electrodes, an insulator, and the skin are vertically arranged to detect whether the wearable device touches the skin.

A symbol is printed on the shielding layer. The symbol includes at least of an device identification, a production code, and a wearing direction.

The electrode unitfurther includes a base layer, and both the top and bottom surfaces of the base layer is covered by a copper foil conductive layer.

Other aspects, features, and advantages other than those described above will become apparent from the detailed description, claims and drawings for carrying out the following disclosure.

The wearable device according to an embodiment of the present disclosure has a structure that is highly flexible in response to the skin surface, enhancing adhesion and allowing accurate measurement of bio-signals.

In addition, the wearable device includes the shielding layer made of the material and/or structure blocking noise, such as static electricity, generated by the user's clothing, thereby preventing noise during measurement, displaying an identification number or the attachment direction of the wearable device, and displaying diffused reflection or specific symbols.

Other aspects, features, and advantages other than those described above will become apparent from the detailed description, claims and drawings for carrying out the following disclosure.

### BRIEF DESCRIPTION OF THE DRAWINGS

FIG. 1A is a front view of the wearable device 10 according to embodiments of the present disclosure. FIG. 1B is a perspective view of the wearable device 10 according to embodiments of the present disclosure, and FIG. 1C is an exploded view of the wearable device 10 according to embodiments of the present disclosure.
FIG. 2 is a view illustrating a state in which a housing is removed from the wearable device.
FIG. 3 is a view illustrating a shielding layer of the wearable device.
FIG. 4 is a view illustrating a bottom layer of the wearable device.
FIG. 5 illustrates a cross-section of the wearable device taken along a line III-III of FIG.1B;
FIG. 6A is a cross-sectional view of the wearable device.
FIG. 6B is a cross-sectional view of the wearable device including a support member.
FIG. 7 is a view illustrating a state in which the wearable device is attached to a user's body.
FIG. 8 is a view of a network environment where the wearable device is connected to an external user terminal.

### DETAILED DESCRIPTION

Reference will now be made in detail to embodiments, examples of which are illustrated in the accompanying drawings, wherein like reference numerals refer to like elements throughout. In this regard, the embodiments of the present disclosure may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

The disclosure may have various modifications and various embodiments, and specific embodiments are illustrated in the drawings and are described in detail in the detailed description. However, this is not intended to limit the disclosure to particular embodiments, and it will be understood that all changes, equivalents, and substitutes that do not depart from the spirit and technical scope of the disclosure are encompassed in the disclosure. In the description of the disclosure, even though elements are illustrated in other embodiments, like reference numerals are used to refer to like elements.

Hereinafter, embodiments of the disclosure will be described in detail with reference to the accompanying drawings, and in the following description with reference to the drawings, like reference numerals refer to like elements and redundant descriptions thereof will be omitted.

Although the terms "first," "second," etc. may be used to describe various elements, these elements should not be limited by these terms. These terms are only used to distinguish one element from another.

An expression used in the singular encompasses the expression of the plural, unless it has a clearly different meaning in the context.

It will be understood that the terms "comprise," "comprising," "include" and/or "including" as used herein specify the presence of stated features or elements but do not preclude the addition of one or more other features or elements.

Sizes of elements in the drawings may be exaggerated or reduced for convenience of explanation. In other words, because sizes and thicknesses of elements in the drawings are arbitrarily illustrated for convenience of explanation, the disclosure is not necessarily limited thereto.

The x-axis, the y-axis and the z-axis are not limited to three axes of the rectangular coordinate system, and may be interpreted in a broader sense. For example, the x axis, the y axis, and the z axis may be perpendicular to one another or may represent different directions that are not perpendicular to one another.

In the case where a certain embodiment may be implemented differently, a specific process order may be performed in the order different from the described order. As an example, two processes that are successively described may be substantially simultaneously performed or performed in the order opposite to the order described.

The terms used herein are only used to describe particular embodiments and are not intended to limit the scope of the disclosure. It will be understood that the terms "comprise," "comprising," "include" and/or "including" as used herein specify the presence of stated features, numbers, steps, operations, elements, parts, and combinations thereof, but do not preclude in advance the presence or addition of one or more other features, numbers, steps, operations, elements, parts, combinations thereof.

According to an embodiment of the disclosure, the wearable device has a structure for preventing noise caused by static electricity, the structure preventing external static electricity from affecting a main body unit, and thus, the measurement precision of the wearable device may be prevented from deteriorating due to external static electricity.

According to an embodiment of the present disclosure, a wearable device 10 may be an electronic device that is used by being attached to a user's back, chest, shoulder, arm, or leg. For example, according to an embodiment of the disclosure, the wearable device 10 may be attached to a user's skin to measure bio-signals, such as electroencephalography EEG, electromyography EMG, electrocardiography ECG, electrooculography EOG, respiration signal, or blood pressure signal. In an embodiment, the wearable device 10 may detect electrical signals, such as voltage and current impedance (e.g., capacitance and resistance) changes associated with muscle movement, eye movement, brain waves, heartbeat, respiration, or body movement, through sensing electrodes.

The wearable device 10 may be a device which measures electroencephalogram (EEG) by receiving electrical signals, which are generated when neural signals are transmitted between brain neurons, through sensing electrodes. Alternatively, the wearable device 10 may be a device which measures electromyogram (EMG) by receiving electrical signals, which are generated from skeletal muscles, through the sensing electrodes. The wearable device 10 may be a device which measures eye position and movement by receiving electrical signals corresponding to the potential difference generated when the eyes move or a device which detects impedance through respiration and body movements.

Additionally, the wearable device 10 may be a continuous glucose monitor (CGM) utilizing iontophoresis or a transdermal therapeutic device based on measured bio-signals such as electrocardiogram, electroencephalography, electromyography, or electrooculography.

The wearable device 10 according to embodiments of the present disclosure includes an electrode unit in which a sensor for measuring bio-signals is embedded, and has a pattern provided on the top surface of the electrode unit for diffused reflection. The pattern for diffused reflection may include a mesh, a polygonal shape e.g., hexagonal, or a hole array.

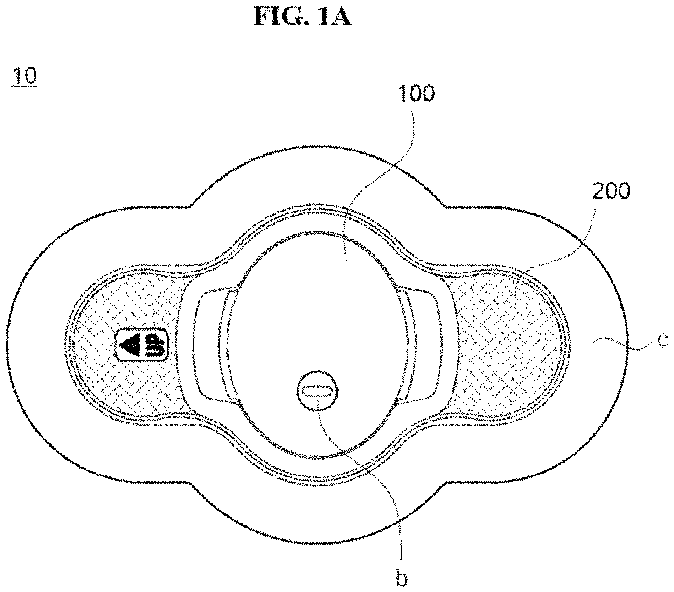

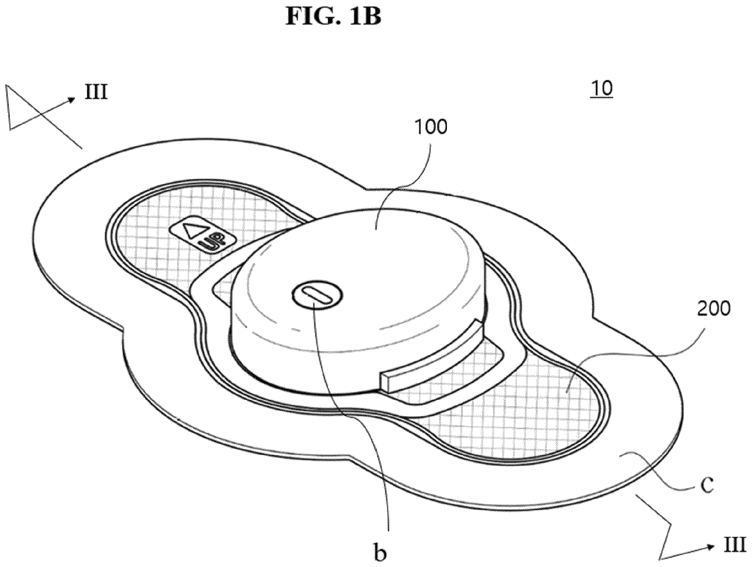

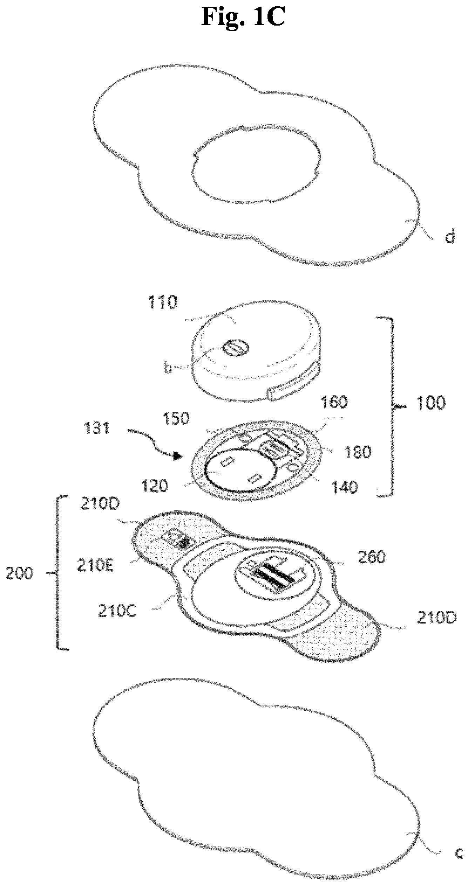

FIG. 1A is a front view of the wearable device 10 according to embodiments of the present disclosure. FIG. 1B is a perspective view of the wearable device 10 according to embodiments of the present disclosure, and FIG. 1C is an exploded view of the wearable device 10 according to embodiments of the present disclosure.

The wearable device 10 may include a main body unit 100 and an electrode unit 200. The electrode unit 200 may be made of a flexible printed circuit board.

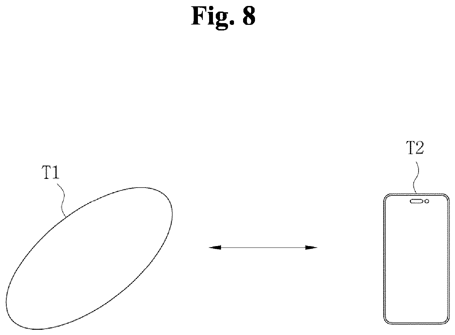

The main body unit 100 is positioned on one side of the wearable device 10 and, as illustrated in FIG. 8b, may include a housing 110, a battery 120, and a processing unit 131. In addition, the main body unit 100 may further include components such as a processor, a memory, a display, a communication module, an indicator, and other support structures for measuring electroencephalography, electromyography, electrocardiogram, and electrooculography while the wearable device 10 is attached to the user's body. Hereinafter, the present disclosure will be focused on specific configurations for the convenience of explanation, but unless otherwise stated, any undescribed components are not excluded from the wearable device 10.

The housing 110 protects other components of the main body unit 100 from external impact and prevents foreign substances from entering. The shape, size, and material of the housing 110 are not particularly limited, and may be properly selected based on the intended use of the wearable device 10. In one embodiment, the housing 110 may have a dome shape with an internal space.

A button b may be positioned on the top surface of the housing 110. Although FIG. 1A illustrates a single power button on the housing 110, the present disclosure is not limited thereto. For example, a plurality of buttons for controlling power and operation modes of the wearable device 10 may be placed on the top or side surfaces of the housing 110.

The battery 120 and the processing unit 131 may be arranged in the internal space of the housing 110. For example, as illustrated in FIG. 9, the processing unit 131 may include a signal amplification circuit, a signal processing circuit, a control circuit, a processor, and a memory for operating the wearable device 10. The processing unit 131 can acquire and process signals measured from the sensing electrodes. The processing unit 131 may include a built-in signal processing module. The signal processing module can process signals using algorithms generated from previously measured signals. The signal processing module can determine whether an electrical value of the signal is within a normal range and can transmit and output the determined information to a connected electronic device. The signal processing module can also indicate whether a signal is being measured or not and transmit the information to the connected electronic device.

As illustrated in FIG. 8c, the wearable device 10 may further include an adhesive layer provided on another side of the electrode unit 200 and an attachment sheet c to cover the adhesive layer. As occasion demands, the wearable device 10 may be manufactured without the attachment sheet c. When the wearable device 10 is in use, the attachment sheet c can be removed.

During use, the attachment sheet c is peeled off, and the wearable device 10 is attached to the user's body. The attachment sheet c may be made of plastic vinyl material such as polyvinyl chloride but is not limited to the material. Alternatively, the wearable device 10 may be manufactured without the attachment sheet c and may be attached to the attachment sheet c after production.

In another embodiment, a waterproof sheet d may cover and protect the electrode unit 200. The waterproof sheet d may include an accommodation groove that accommodates the main body unit 100. Both sides of the accommodation groove may cover a portion of the electrode unit 200. If necessary, the wearable device 10 may be manufactured and used without the waterproof sheet d.

In one embodiment, the waterproof sheet d may have a larger surface area than the electrode unit 200. As illustrated in FIG. 1C, the waterproof sheet d may be positioned over the electrode unit 200, with at least a portion of the waterproof sheet protruding outward from the electrode unit 200 to be adhered to the user's skin.

In one embodiment, the waterproof sheet d may be made of a moisture-permeable material. For example, the waterproof sheet d may be a moisture-permeable polyurethane tape or a material with moisture directionality, such as Gore-Tex. Additionally, the waterproof sheet d may be made of a waterproof material. Accordingly, the waterproof sheet d prevents external moisture from entering the wearable device 10 (waterproofing) and allows internal moisture to be released to the outside (moisture permeability).

Hereinafter, a method for manufacturing a wearable device according to an embodiment of the present disclosure will be described.

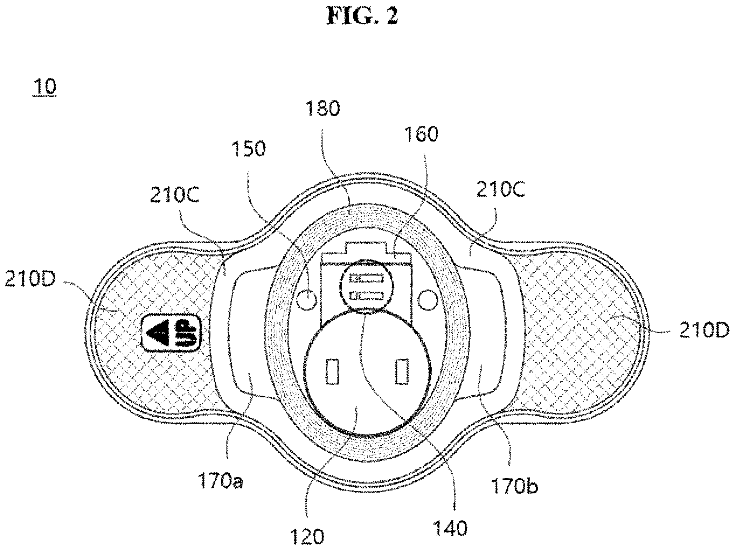

The method for manufacturing a wearable device according to an embodiment of the present disclosure includes assembling a main body unit 100 onto a premanufactured electrode unit 200. An attachment sheet c is attached to the assembled main body unit 100 and one side of the electrode unit 200 to complete the wearable device 10. The wearable device 10 may be delivered to a user as a set that includes a separately manufactured waterproof sheet d. As illustrated in FIG. 2, the main body unit 100 may include an O-ring portion 180 which is in close contact with the housing 110 and a connector 140 to which the electrode unit is adhered. The connector of the electrode unit may pass through a slit 160 and be attached to the connector 140.

The main body unit 100 may include the O-ring portion 180 made of an elastic material corresponding to the housing 110. The O-ring portion 180 may be composed of materials such as silicone, EPDM, NBR, or Viton.

The main body unit 100 may also include coupling portions 170a and 170b. The coupling portions 170a and 170b coupled with the housing may be fastening clips such as hooks.

In an additional embodiment, the main body unit 100 may further include a temperature sensor 150 for detecting temperature. The temperature sensor 150 may detect external temperature.

The electrode unit 200 may incorporate one or more sensing electrodes and electric circuits, and include a base layer and conductive layers provided on both upper and lower surfaces of the base layer. The base layer is formed as a thin sheet made of materials such as polyimide or polyester, and one of the conductive layers arranged on the upper and lower surfaces may include the sensing electrode and the electric circuit.

The electrode unit 200 may further include a shielding layer 210 and a bottom layer 250. The shielding layer 210 may be formed by covering a conductive material with a predetermined material. The shielding layer 210 may be floating relative to the main body unit 100. That is, the shielding layer 210 and the main body unit 100 may maintain a state not electrically connected to each other and may have a floating potential.

The shielding layer 210 may include a region 210C where the main body unit 100 is positioned and a region 210D where the main body unit 100 is not positioned. The region 210D where the main body unit 100 may extend outward in both directions from the main body unit 100, but is not limited thereto and may extend in only one direction among left, right, top, and bottom directions.

Preferably, the electrode unit 200 may have a single sensing electrode in the center and one additional sensing electrode positioned in each direction. However, the locations of the sensing electrodes may vary depending on the placement of the region 210D where the main body unit 100 is not positioned.

The region 210C where the main body unit is positioned and the region 210D where the main body unit is not positioned may be arranged in a row. In another embodiment, the region 210C where the main body unit is positioned and the region 211D where the main body unit is not positioned may be arranged at a predetermined angle.

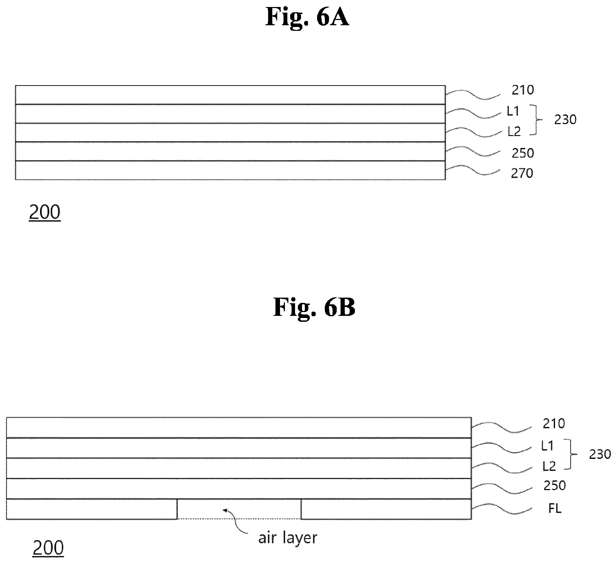

According to embodiments of the present disclosure, the shielding layer 210 of the electrode unit 200 is covered with an opaque material to prevent exposure of the sensing electrodes and/or the electric circuits. L1 and L2 in FIG. 6A are a three-layer electric circuit layers in case of a two-layer structure.

The electrode unit 200 of the wearable device 10 may have a base layer made of polyimide or polyester. The electrode unit 200 may further include conductive layers containing the sensing electrodes and the electric circuits that transmit electrical signals from the sensing electrodes, and an insulating layer for electrical isolation of the conductive layers from external influences.

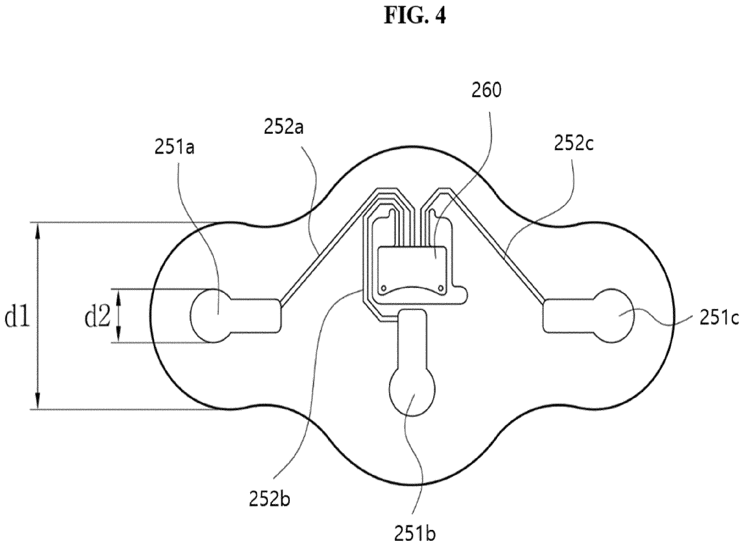

In embodiments of the present disclosure, additional insulating layers may be formed on the shielding layer 210 and the bottom layer 250. The electric circuits of the electrode unit 200 are connected to the main body unit 100, performing functions such as receiving or transmitting electrical signals. Additionally, the electrode unit 200 may include a connector 260 (see FIG. 4) to be connected with the processing unit 131 of the main body unit 100.

The electrode unit 200 may include a base layer 230, a shielding layer 210, and a bottom layer 250. The base layer 230 may comprise a film and a conductive layer made of conductive material. The shielding layer 210 and the bottom layer 250 may include the conductive layers made of conductive material and/or insulating layers. The shielding layer 210 may be formed by coating the conductive layer with a predetermined material. The bottom layer 250 is positioned on the bottom surface of the base layer 230, and the shielding layer 210 and the bottom layer 250 may be made of different materials. The shielding layer 210 may be formed of a first material, and the bottom layer 250 may be formed of a second material.

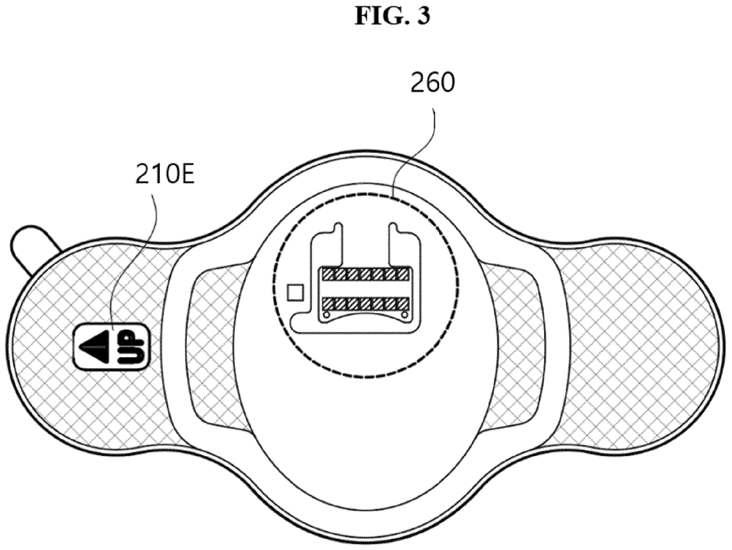

As illustrated in FIG. 3, the shielding layer 210 may have a first pattern in the region 210C where the main body unit 100 is positioned to enhance adhesion between the shielding layer and the main body unit arranged on one side of the flexible printed circuit board. The first pattern may be formed to correspond to the contact surface of the main body unit. For example, the first pattern may have a smooth surface matching the contact surface of the main body unit. In another embodiment, the first pattern may be formed to conform to the contact surface of the main body unit. The first pattern may be a solid pattern, but is not limited thereto and may include similar patterns.

In another embodiment, on the shielding layer 210, the first pattern is formed in a region where the boundary of the main body unit 100 is located, and a pattern different from the first pattern may be formed in the region where the main body unit 100 is located.

The shielding layer 210 may have one or more second patterns formed on the entire or a portion of the region where the main body unit 100 is not positioned. The second pattern may be different from the first pattern and have a predetermined shading effect on the surface. Thus, the shielding layer 210 increases flexibility in the region where the main body unit 100 is not positioned, thereby enhancing adhesion to the user's skin. FIG. 2 illustrates an example where the first pattern 210C and the second pattern 210D are applied. The second pattern may be one of a mesh, a polygonal shape (e.g., hexagonal), and a hole array. To enhance the diffused reflection effect, the second patterns may be formed with a predetermined minimum spacing. The minimum spacing value may be, for example, 2 to 3 mm.

Due to the second pattern formed on the shielding layer 210, the electrode unit can be bent in close contact with the skin. For instance, the second pattern may be a mesh pattern where a predetermined pattern is repeated. The second pattern may include a plurality of mesh patterns. The second pattern formed on the shielding layer 210 enables the electrode unit to achieve diffused reflection of external stimulation. The second pattern formed on the shielding layer 210 is covered over the conductive layer, achieving diffused reflection of external stimulation.

The shielding layer 210 may be covered with an opaque material to prevent visual exposure of the electrodes and electric circuits of the electrode unit. The shielding layer 210 having the opaque-colored material and the specific pattern allows diffused reflection of external light, and can block external electromagnetic waves, static electricity, and impedance changes caused by external body contact.

The shielding layer 210 may be formed by coating or laminating a first material. Paint and materials of the first material may be covered (laminated or coated) over the conductive layer of the conductive material to form the shielding layer 210. The thickness of the shielding layer 210 may vary between the region 210C where the main body unit 100 is positioned and the region 210D where the main body unit 100 is not positioned. For example, the thickness of the region 210C where the main body unit 100 is positioned may be greater than that of the region 210D where the main body unit 100 is not positioned.

The shielding layer 210 may include a label 210E. The label 210E may include an identification number assigned to each wearable device 10, a designation number for a target where the wearable device 10 is to be attached. The label 210E may further include information related to an attachment direction(wearing direction). the shielding layer 210 may include at least of specific symbols, device identification information, and directional information.

The bottom layer 250 is positioned in a direction that the electrode unit 200 is attached to the skin and may be formed with a second material coating. While the shielding layer 210 is formed of the first material, the bottom layer 250 is formed of the second material. The second material may be transparent, unlike the first material, but is not limited thereto and may also be made of an opaque material. The bottom layer 250 may be formed from an electrically insulating material.

Unlike the shielding layer 210, the entire bottom layer 250 may be formed with a single pattern. The bottom layer 250 with the single pattern can enhance adhesion to the user's skin.

In another embodiment, an additional support including an opening may be arranged on one side of the bottom layer 250. Due to the additional support, an air layer may be formed between the support and the skin. The opening may be positioned to correspond with the sensing electrode. When the additional support is present, the sensing electrode can detect changes of the air layer as electrical signals.

The sensing electrode may be designed to detect electro-physical signals. The sensing electrode can detect electrical signals from the body, including the skin, eyes, muscles, and brain waves. The sensing electrode can detect changes in the air layer formed between the skin and the sensing electrode as electrical signals. For example, when the user's body is changed depending on the user's breathing or movement, the air layer surrounding the sensing electrode. These changes in the air layer formed on the sensing electrode is changed, and the change in the air layer can be detected as variations in electrical signals. The signals detected by the sensing electrode may include ECG, EMG, EOG, and impedance. The sensing electrode can also measure capacitance values.

The bottom layer 250 has a uniform thickness to enhance contactability with the body. Through the bottom layer 250, the sensing electrode can detect electrical signals generated by muscle movement, heart activity, eye movement, and brain waves.

FIG. 4 is a rear view of the electrode unit 200 of the wearable device.

As illustrated in FIG. 4, the electrode unit 200 may include three sensing electrodes 251a, 251b, and 251c. The bottom layer 250 of the wearable device may be formed from transparent materials, coatings, or substances, allowing visual exposure of the electrodes. Through the bottom layer 250, the sensing electrodes and the electrical circuits may be exposed visually.

The sensing electrodes 251a, 251b, and 251c can measure potential differences associated with heartbeat, muscle movement, eye movement, and brain waves on the body surface, or apply current to administer electrolyte-based drugs into the body through the user's skin or mucous membranes. The sensing electrodes can also be used to measure impedance between the electrodes.

The region where the sensing electrode 251a is positioned on the electrode unit 200 may be larger than the sensing electrodes 251a, 251b, and 251c. For example, the region d2 where the sensing electrodes 251a, 251b, and 251c are arranged may be smaller than d1 and may be approximately one-third the size of d1. The size of d1 may be twice or three times that of d2, but is not limited thereto and may vary.

As illustrated in FIG. 4, an adhesive layer 270 having adhesion may be further provided on one side of the wearable device. The adhesive layer 270 may be positioned on one side of the bottom layer 250 and may be composed of silicone-based adhesives, polyacrylate adhesives, hydrogel, or similar materials. The adhesive layer 270 may be formed to cover all or a portion of the bottom layer 250.

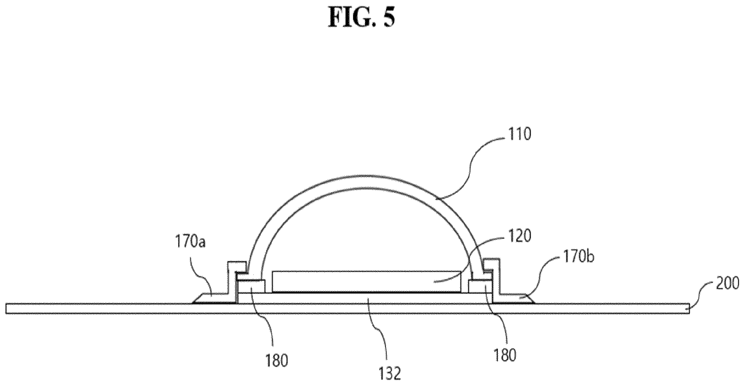

FIG. 5 illustrates a cross-section of the wearable device taken along a line III-III of FIG.1B.

The method for manufacturing a wearable device 10 can manufacture a wearable device 10 by stacking multiple layers to manufacture an electrode unit 200 as a flexible circuit board, combining the electrode unit 200 of the manufactured flexible circuit board with a processing unit 131, and then assembling the housing 110. The cross-section of the assembled wearable device 10 is as shown in FIG. 5. More specifically, the electrode unit 200 of the flexible circuit board and the connector 140 of the processing unit 131 are combined, so that the electrode unit 200 and the processing unit 131 can be combined. The processing unit 131 may include the battery 120, the connector 140, the slit 160, the coupling portion 170a, 170b, and the O-ring portion 180. The processing unit 131 may further include a substrate 132 on which the connector 140, the slit 160, the coupling portion 170a, 170b, and the O-ring portion 180 are arranged. The O-ring portion 180 may be arranged along an edge of the substrate 132. When the housing 110 is pressed to be arranged onto the O-ring portion 180, the housing 110 may be attached to the substrate 132 by the coupling portion 170a, 170b.

FIG. 6A is a cross-sectional view of the electrode unit 200. As illustrated in FIG. 6A, the base layer 230 may include a film layer L1, and a conductive layer L2 made of a conductive material, and further include a shielding layer 210 provided on the upper portion of the film layer and a bottom layer 250 provided on the lower portion of the film layer. An adhesive layer 270 may be formed on the lower portion of the bottom layer 250. The adhesive layer 270 may be formed on the bottom layer 250, allowing the device to attach to the user's skin.

FIG. 6B is a cross-sectional view of the electrode unit 200. As illustrated in FIG. 6B, the base layer 230 of the wearable device 10 may include a film layer L1 and a conductive layer L2, which includes sensing electrodes and electrical circuits. The electrode unit 200 may further include a shielding layer 210 provided on the upper portion of the film layer, a bottom layer 250 provided on the lower portion of the film layer, and a support member FL provided next to the sensing electrode, such that an air layer is formed below the sensing electrode. The height of the support member FL may preferably be between 1 um and 5 um. The air layer allows the patch device to be attached to the skin to detect capacitance changes caused by the skin movement between the skin and the electrodes of the FPCB, enabling the measurement of respiratory signals.

FIG. 6A illustrates a two-layer film, wherein the thickness of the film layer L1 is preferably 2 um, and the thickness of the conductive layer L2 is also 12 um. The shielding layer 210 and the bottom layer 250 may be formed thinner than the film layer L1 and conductive layer L2, for example, around 10 um.

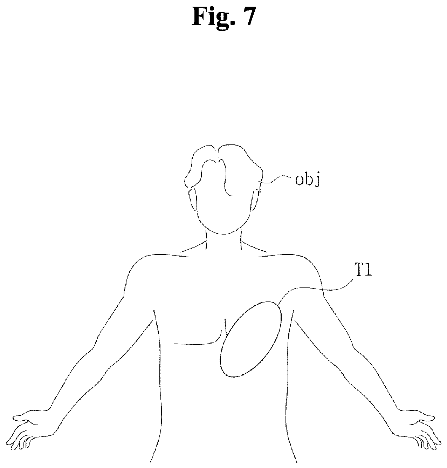

FIG. 7 is a view illustrating the network environment of a wearable device T1 according to the embodiments of the present invention.

As illustrated in FIG. 7, the wearable device T1 may be mounted non-invasively or invasively near the heart of a subject obj to detect electrocardiographic (ECG) signals. The subject obj may refer to a human, an animal, or a body part of the human or animal, such as the chest, scalp, arms, or legs, but is not limited thereto.

The wearable device T1 may also be attached to body areas such as the scalp, and muscle regions, or around the eyes to measure electroencephalography (EEG), electromyography (EMG), and electrooculography (EOG). The wearable device T1 can be attached to a body part depending on the type of bio-signals to be measured, measuring bio-signals. The wearable device T1 can receive the measured electrocardiogram signal and process the electrocardiogram signal using a predetermined method. The wearable device T1 may store and execute a program related to signal processing.

FIG. 8 is a view of a network environment where the wearable device is connected to an external user terminal.

The wearable device T1 may transmit and receive data with a user terminal T2 using a communication module built into the processing unit. The communication module may include various wireless communication modules, such as a wireless internet module, a short-range communication module, and a mobile communication module.

The wireless internet module means a module performing communication with external networks based on protocols such as Wireless LAN (WLAN), Wi-Fi, Wireless Broadband (WiBro), Worldwide Interoperability for Microwave Access (WiMAX), and High-Speed Downlink Packet Access (HSDPA).

The short-range communication module means a module for performing communication with nearby external devices using short-range communication methods, such as Bluetooth, Radio Frequency Identification (RFID), Infrared Data Association (IrDA), Ultra Wideband (UWB), and ZigBee.

The mobile communication module means a module for performing communication with a mobile communication network following various cellular standards such as 3rd Generation (3G), 3rd Generation Partnership Project (3GPP), and Long-Term Evolution (LTE).

However, the communication module is not limited to the technologies, and any other type of communication module capable of transmitting and receiving signals and data with the wearable device T1 may be implemented.

The wearable device T1 may include one or more measurement electrodes for measuring bio-signals such as EEG, EMG, ECG, EOG, respiration signal, and blood glucose levels. The wearable device T1 may store the measured bio-signals in an internal memory. The wearable device T1 can also transmit the bio-signal data to an external device, such as an ECG signal processing unit, through the communication module. Additionally, the wearable device T1 may transmit the measured bio-signals in real time to a user terminal T2.

The user terminal T2 may process the bio-signals measured from the subject obj using methods specific to each bio-signal type. The user terminal T2 can divide the measured bio-signals into predefined signal segments and group or cluster the signal segments. The user terminal T2 may group or cluster each reference signal segment and signal segments with similar patterns based on one or more reference signal segments. The user terminal T2 may also group or cluster the signal segments based on one or more reference values.

An authorized user, such as a medical professional or an analyst, can access the bio-signals through the user terminal T2. The user terminal T2 communicates with the wearable device T1 to receive and display the measured or ongoing bio-signal data.

A patient carrying the user terminal T2 can monitor the bio-signals in real time. The wearable device T1 can transmit a value indicating whether the bio-signal is being properly measured to the user terminal T2. If the bio-signal is not being measured correctly, the wearable device T1 can send a value indicating whether the adhesion of the wearable device T1 to the skin gets weakened, whether displacement of the wearable device affects on the measurement of the bio-signals, and whether the measured bio-signals are within a normal range. In case of abnormal measurement, the wearable device T1 transmits specific values indicating different causes of measurement failure.

The wearable device T1 may output values of the measurement status through a separate output unit. The wearable device T1 may include an output unit, such as an LED to display the measurement status.

As described above, the disclosure has been described with reference to the embodiment illustrated in the drawings, but this is merely an example. Those of ordinary skill in the art will fully understand that various modifications and other equivalent embodiments can be made from the embodiments. Therefore, the scope of the protection of the technology of the disclosure should be determined by the appended claims.

Specific technical descriptions in the embodiments are embodiments and do not limit the technical scope of the embodiments. In order to concisely and clearly describe the disclosure, descriptions of general techniques and configurations of the related art may be omitted. Also, connections or connection members of lines between elements illustrated in the drawings are examples of functional connections and/or physical or circuit connections, and may be represented by various alternative or additional functional connections, physical connections, or circuit connections in an actual device. In addition, unless specifically stated as "essential" or "importantly", an element may not be a necessary element for the application of the disclosure.

The term "above" or similar referring expressions used in the description and claims of the disclosure may refer to both the singular and plural expressions unless otherwise specified. Also, when a range is described in the embodiments, it means that embodiments to which individual values belonging to the range are applied are also included (unless otherwise stated), it is the same as each individual value constituting the range is described in the detailed description of the disclosure. Moreover, steps or operations constituting the method according to the embodiments may be performed in an appropriate order, if the order is explicitly stated or unless otherwise stated. The embodiments are not necessarily limited according to the order of the description of the steps or operations. All examples or illustrative terms (e.g., etc.) in the embodiments are merely used to describe the embodiments in detail, and the scope of the embodiments is limited by the examples or illustrative terms unless limited by the claims. In addition, those of ordinary skill in the art will appreciate that various modifications, combinations, and changes can be made in accordance with design conditions and factors within the scope of the appended claims or equivalents thereof.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope included in the following claims.

## Claims

1. A wearable device comprising:
a main body unit which includes a housing and a processing unit arranged within the housing; and
an electrode unit which is electrically connected to the main body unit, includes at least one sensing electrode and electrical circuits for the at least one sensing electrode, and is formed of a conductive member,
wherein the electrode unit includes a shielding layer having a predetermined pattern formed in a certain region and a bottom layer getting in contact with a user's skin, and
wherein the shielding layer is laminated or coated with a predetermined pattern made of an opaque material to diffuse external noise.

2. The wearable device according to claim 1, wherein the shielding layer includes a first region where the main body unit is positioned and a first pattern is formed, and a second region in which a second pattern different from the first pattern is formed.

3. The wearable device according to claim 1, wherein the shielding layer includes a region where the main body unit is not positioned and a predetermined pattern is formed.

4. The wearable device according to claim 2, wherein the first pattern formed on the shielding layer is a solid pattern that increases bonding strength with the main body unit.

5. The wearable device according to claim 2, wherein the second pattern formed on the shielding layer is a mesh pattern that reduces external signals including static electricity caused by a user's clothing.

6. The wearable device according to claim 1, wherein an electrode that senses electro-physical signals is arranged in an opening of the bottom layer.

7. The wearable device according to claim 1, wherein an electrode that detects capacitance is arranged in the opening of the bottom layer.

8. The wearable device according to claim 6, wherein one of the sensing electrode, an insulator, and an air layer formed in the opening of the bottom layer are vertically arranged,
the air layer which is a gap with the skin, and
the one of sensing electrodes detects a change in a depth of the air layer as a change in capacitance value due to skin movement.

9. The wearable device according to claim 6, wherein one of the sensing electrodes, an insulator, and the skin are vertically arranged,
the one of sensing electrodes detect whether the wearable device touches the skin by sensing a capacitance value.

10. The wearable device according to claim 1, wherein a symbol including at least of an device identification, a production code, and a wearing direction is printed on the shielding layer.

11. The wearable device according to claim 1, wherein the electrode unit further includes a base layer, and both a top and bottom surfaces of the base layer is covered by a copper foil conductive layer.
